(19) Europäisches Patentamt
European Patent Office
Office européen des brevets

(11) Veröffentlichungsnummer: **0 027 954**
**B1**

(12) # EUROPÄISCHE PATENTSCHRIFT

(45) Veröffentlichungstag der Patentschrift: **24.11.82**

(51) Int. Cl.³: **C 07 C125/065**, C 07 C125/073

(21) Anmeldenummer: **80106250.6**

(22) Anmeldetag: **15.10.80**

(54) **Verfahren zur Herstellung von Urethanen und ihre Verwendung zur Herstellung von Isocyanaten.**

(30) Priorität: **27.10.79 DE 2943549**

(43) Veröffentlichungstag der Anmeldung: **06.05.81 (Patentblatt 81/18)**

(45) Bekanntmachung des Hinweises auf die Patenterteilung: **24.11.82 Patentblatt 82/47**

(84) Benannte Vertragsstaaten: **AT BE CH DE FR GB IT LI NL SE**

(56) Entgegenhaltungen:
**US A 3 950 285**

**JOURNAL OF ORGANIC CHEMISTRY, Band 18, 1953 Baltimore N.G. GAYLORD et al. « The Reactions of Carbamates with Alcohols » Seiten 1 632 bis 1 637**

(73) Patentinhaber: **BAYER AG**
**Zentralbereich Patente, Marken und Lizenzen**
**D-5090 Leverkusen 1, Bayerwerk (DE)**

(72) Erfinder: **Findeisen, Kurt, Dr.**
**In der Follmuehle 10**
**D-5068 Odenthal 2 (DE)**
Erfinder: **König, Klaus, Dr.**
**Heymannstrasse 50**
**D-5090 Leverkusen 1 (DE)**
Erfinder: **Fauss, Rudolf, Dr.**
**Gerstenkamp 10**
**D-5000 Koeln 80 (DE)**
Erfinder: **Heitkämper, Peter, Dr.**
**Fliederweg 14**
**D-4047 Dormagen 11 (DE)**

**EP 0 027 954 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung von Urethanen und ihre Verwendung zur Herstellung von Isocyanaten

Die vorliegende Erfindung betrifft ein neuartiges Verfahren zur Herstellung von Urethanen, die als Ausgangsmaterialien zur Herstellung von Isocyanaten durch thermische Spaltung besonders gut geeignet sind, sowie ihre Verwendung zur Herstellung von Isocyanaten.

Die phosgenfreie Herstellung von Urethanen und deren anschließende thermische Spaltung in die entsprechenden Isocyanate stellt eine wichtige Alternative zur an sich bekannten Herstellung von Isocyanaten durch Phosgenierung der entsprechenden primären Amine dar. Gemäß US-PS 2 409 712 bzw. US-PS 2 806 051 erfolgt diese phosgenfreie Urethansynthese durch Umsetzung von Harnstoff mit primären Aminen und Alkoholen oberhalb 100 °C. Wie diesen Veröffentlichungen zu entnehmen und wie auch durch eigene Versuche der Anmelderin bestätigt wurde, ist bei der Durchführung dieser Synthese die Anwendung von Temperaturen von ca. 140 bis 200 °C zwecks Erzielung maximaler Ausbeuten besonders sinnvoll. Im Falle der Verwendung von unter diesen Temperaturbedingungen siedenden Alkoholen ist daher ein Arbeiten unter Druck unerläßlich. Andererseits führt ein derartiges Arbeiten unter Druck ganz abgesehen von dem damit verbundenen erhöhten apparativen Aufwand zu der nachteilhaften Begleiterscheinung, daß das sich bildende Ammoniakgas nicht mehr sofort nach seiner Entstehung entweichen kann, sondern sich vielmehr in dem Reaktionsprodukt löst. Dies hat wiederum einen vorzeitigen Stillstand der Umsetzung und somit schlechtere Ausbeuten zur Folge. Man wird daher in der Praxis bestrebt sein, die Synthese unter Verwendung von vergleichsweise hochsiedenden Alkoholen durchzuführen, so daß sich die Anwendung eines äußeren Drucks erübrigt. Andererseits ist die Verwendung von vergleichsweise hochsiedenden Alkoholen, d.h. von Alkoholen, deren Siedepunkt in erster Näherung dem Siedepunkt des dem Urethan entsprechenden Isocyanats entspricht, mit dem Nachteil verbunden, daß bei der anschließenden thermischen Spaltung Spaltprodukte (Isocyanat und Alkohol) erhalten werden, die sich wegen ihres gleichen oder nur geringfügig verschiedenen Siedeverhaltens schwer destillativ trennen lassen. Auf eine sofortige und wirkungsvolle Trennung der Spaltungsprodukte zwecks Vermeidung ihrer Rekombination kommt es jedoch bei der Herstellung der Isocyanate durch thermische Spaltung der Urethane entscheidend an.

Aufgabe der vorliegenden Erfindung war es daher, ein Verfahren zur Verfügung zu stellen, welches die Modifizierung von Urethanen auf Basis von oberhalb 140 °C siedenden Alkoholen gestattet, so daß Urethane erhalten werden, die leicht thermisch in das entsprechende Isocyanat und den entsprechenden Alkohol gespalten werden können, und deren Spaltprodukte einen zwecks ihrer sofortigen destillativen Auftrennung ausreichend unterschiedlichen Siedepunkt aufweisen.

Wie überraschend gefunden wurde, konnte diese Aufgabe dadurch gelöst werden, daß man die genannten Urethane bei erhöhter Temperatur mit einem unter 140 °C siedenden Alkohol umsetzt. Die Durchführbarkeit einer derartigen « Umurethanisierung » ist überraschend, da eine schwerer flüchtige Komponente (höhersiedender Alkohol) durch eine leichter flüchtige Komponente (niedriger siedender Alkohol) ersetzt werden muß. Aus der vorveröffentlichten Literatur ist lediglich die plausible umgekehrte Umurethanisierung bekannt (J. Organic Chemistry 18 (1953), S. 1632-1633 und US-PS 3 950 285).

Gegenstand der vorliegenden Erfindung ist somit ein Verfahren zur Herstellung von als Ausgangsmaterial zur Herstellung von organischen Isocyanaten durch thermische Spaltung geeigneten Urethanen der Formel

$$R_1\,(NHCOOR_3)_n$$

in welcher

$R_1$ für einen durch Entfernung der Isocyanatgruppen aus einem n-wertigen organischen Isocyanat mit einem unter Normaldruck oberhalb 100 °C liegenden Siedepunkt erhaltenen organischen, unter den Reaktionsbedingungen inerten Rest,

$R_3$ für einen durch Entfernung der Hydroxylgruppe aus einem unter Normaldruck zwischen 60 und 140 °C siedenden einwertigen Alkohol erhaltenen, unter den Reaktionsbedingungen inerten Rest steht, wobei das dem Rest $R_1$ zugrundeliegende Isocyanat $R_1\,(NCO)_n$ unter Normaldruck einen mindestens 30 °C höheren Siedepunkt aufweist als der dem Rest $R_3$ entsprechende Alkohol $R_3OH$, und

n für eine ganze Zahl von 1 bis 3 steht,

dadurch gekennzeichnet, daß man Urethane der Formel

$$R_1\,(NHCOOR_2)_n$$

in welcher

$R_1$ und n die bereits genannte Bedeutung haben und

$R_2$ für einen durch Entfernung der Hydroxylgruppe aus einem einwertigen Alkohol mit einem unter Normaldruck oberhalb 140 °C liegenden Siedepunkt erhaltenen, unter den Reaktionsbedingungen inerten Rest steht,

mit einer solchen Menge eines, unter Normaldruck einen mindestens 10 °C unter dem Siedepunkt des dem Urethan zugrundeliegenden Alkohols $R_2$-OH liegenden Siedepunkt aufweisenden, Alkohols der Formel

$$R_3{-}OH$$

bei 120 °C bis 400 °C, gegebenenfalls in Gegenwart von Katalysatoren und gegebenenfalls in Gegenwart von Lösungsmitteln zur Reaktion bringt, daß für jedes Mol des Urethans der Formel $R_1$ ($NHCOOR_2$)$_n$ mindestens n Mole des Alkohols $R_3$—OH zur Verfügung stehen.

Gegenstand der Erfindung ist auch die Verwendung der Verfahrensprodukte als Ausgangsmaterial zur Herstellung von organischen Isocyanaten.

Ausgangsmaterialien für das erfindungsgemäße Verfahren sind beliebige 1 bis 3 N- und O-substituierte, Urethangruppen aufweisende Verbindungen, deren N-Substituenten dem ein- oder mehrwertigen Rest entsprechen, der durch Entfernung der Isocyanatgruppen aus einem mono- oder polyfunktionellen Isocyanat mit einem unter Normaldruck oberhalb 100 °C liegenden Siedepunkt erhalten worden ist, und deren O-Substituenten dem Rest entsprechen, wie er durch Entfernen der Hydroxylgruppe aus einem einwertigen, unter Normaldruck oberhalb 140 °C siedenden Alkohol erhalten worden ist, mit der Einschränkung, daß beide der genannten Substituenten unter den Reaktionsbedingungen des Erfindungsgemäßen Verfahrens inert sind ; sowie beliebige einwertige Alkohole mit einem unter Normaldruck zwischen 60 und 140 °C, mindestens jedoch 10 °C, vorzugsweise mindestens 50 °C unter dem Siedepunkt des dem Urethan zugrundeliegenden Alkohols und mindestens 30 °C unterhalb des Siedepunkts des dem Urethan zugrundeliegenden Isocyanat liegenden Siedepunkt, welche, von der alkoholischen Hydroxylgruppe abgesehen, unter den Bedingungen des erfindungsgemäßen Verfahrens inert sind.

Geeignete Urethane a) sind beispielsweise solche der Formel

$$R_1\,(NHCOOR_2)_n$$

in welcher

$R_1$, $R_2$ und n die bereits oben genannte Bedeutung haben. Für das erfindungsgemäße Verfahren bevorzugte Urethane sind solche der genannten allgemeinen Formel, für welche

$R_1$ für einen gegebenenfalls chlorsubstituierten, vorzugsweise jedoch unsubstituierten aliphatischen Kohlenwasserstoffrest mit 6 bis 18 Kohlenstoffatomen, einen gegebenenfalls Methyl- oder Chlor-Substituenten und/oder gegebenenfalls Methylenbrücken aufweisenden aromatischen Kohlenwasserstoffrest mit 6 bis 15 Kohlenstoffatomen, einen cycloaliphatischen Kohlenwasserstoffrest mit 6 bis 15, insbesondere 6 bis 10 Kohlenstoffatomen oder einen araliphatischen Kohlenwasserstoffrest mit 7 bis 15, vorzugsweise 7 bis 8 Kohlenstoffatomen steht,

$R_2$ für einen gegebenenfalls $C_1$-$C_4$-Alkoxy- oder $C_1$-$C_4$-Alkoxy-$C_2$-$C_4$-alkoxy-substituierten oder unsubstituierten primären oder sekundären aliphatischen Kohlenwasserstoffrest mit 5 bis 18 Kohlenstoffatomen, einen sekundären cycloaliphatischen Kohlenwasserstoffrest mit 6 bis 15 Kohlenstoffatomen, insbesondere einen Cyclohexylrest oder einen primären araliphatischen Kohlenwasserstoffrest mit 7 bis 15 Kohlenstoffatomen, insbesondere 7 bis 8 Kohlenstoffatomen steht und

n für 1 oder 2 steht, wobei im Falle von n = 2 zwischen den beiden Substituenten — $NHCOOR_2$ des Restes $R_1$ mindestens 2 Kohlenstoffatome angeordnet sind.

Typische Beispiele für das erfindungsgemäße Verfahren geeignete Urethane sind N-(n-Hexyl)-O-(n-hexyl)-urethan, N-Stearyl-O-cyclohexylurethan, N-Phenyl-O-cyclohexyl-urethan, N-(p-Totyl)-O-benzyl-urethan, N-(3,4-Dichlorphenyl)-O-(2-phenylethyl)-urethan, 4,4'-Bis-(hexoxycarbonylamino)-diphenylmethan, 2,4-Bis-(Cyclohexoxycarbonylamino)-toluol oder 1-(Cyclohexoxycarbonylamino)-3,5,5-trimethyl-5-(cyclohexoxycarbonyl-amino-methyl)-cyclohexan, d.h. das sich formal aus 1 Mol Isophorondiisocyanat und 2 Mol Cyclohexanol ableitende Bis-urethan.

Beispiele für das erfindungsgemäße geeignete Alkohole b) sind insbesondere solche der Formel

$$R_3\text{—OH}$$

in welcher

$R_3$ die bereits obengenannte Bedeutung hat.

Bevorzugte Alkohole der genannten allgemeinen Formel sind solche, für welche $R_3$ für einen primären oder sekundären aliphatischen Kohlenwasserstoffrest mit einem bis vier Kohlenstoffatomen steht.

Typische Vertreter geeigneter Alkohole der genannten allgemeinen Formel sind z.B. Methanol, Ethanol, n-Propanol, iso-Propanol, n-Butanol oder sec.-Butanol.

Bei der Durchführung des erfindungsgemäßen Verfahrens werden die beispielhaft genannten Ausgangsmaterialien a) und b) in solchen Mengenverhältnissen eingesetzt, daß auf jedes Mol des Urethans a) mindestens n Mole, vorzugsweise 5 n bis 20 n Mole des Alkohols b) zur Verfügung stehen, wobei n die obengenannte Bedeutung hat.

Das erfindungsgemäße Verfahren wird im Temperaturbereich von 120 °C bis 400 °C durchgeführt, wobei die Umsetzung in der Gasphase im Temperaturbereich zwischen 250 °C und 400 °C, insbesondere 280 °C bis 360 °C oder in der Flüssigphase zwischen 120 °C und 250 °C, vorzugsweise 140 °C bis 250 °C und insbesondere zwischen 150 °C und 240 °C erfolgt. Die Umsetzung kann beispielsweise nach einer der folgenden 4 Varianten durchgeführt werden :

1. Das umzusetzende Urethan a) wird in dem niedrig siedenden Alkohol b) gelöst und bei Temperaturen zwischen 250 °C und 400 °C in der Gasphase kontinuierlich zur Umsetzung gebracht. Hierbei gibt man beispielsweise die Lösung der Reaktanten kontinuierlich in ein mit Füllkörpern gefülltes Reaktionsrohr, welches auf die genannte Umsetzungstemperatur erhitzt ist, worauf die Umsetzung nach der spontan erfolgenden Verdampfung der Ausgangslösung im wesentlichen in der Gasphase abläuft. Das am

Austritt des Reaktionsrohrs anfallende, im wesentlichen aus Verfahrensprodukt, überschüssigem Alkohol $R_3$—OH und abgespaltetem Alkohol $R_2$—OH bestehende, gasförmige Gemisch wird beispielsweise in vorgelegtem flüssigem Alkohol $R_3$—OH kondensiert und anschließend wie nachstehend beschrieben aufgearbeitet.

2. Das als Ausgangsmaterial eingesetzte Urethan a) wird in einem mit einem Rührer, einer Destillationsbrücke und einem Einleitungsrohr versehenen Reaktionsgefäß in flüssiger bzw. geschmolzener Form bei 120 °C bis 250 °C vorgelegt. Anschließend erfolgt die allmähliche Zugabe des Alkohols b) ($R_3$—OH) unter stetigem Rühren der auf 120 °C bis 250 °C gehaltenen Vorlage. Das hierbei anfallende Destillat besteht aus überschüssigem, nicht umgesetztem Alkohol $R_3$—OH, abgespaltetenem Alkohol $R_2$—OH und je nach dem Dampfdruck des Verfahrensprodukts aus Verfahrensprodukt. Falls als Verfahrensprodukt ein Urethan entsteht, welches unter den gegebenen Temperaturverhältnissn nicht flüchtig ist, verbleibt dies im Reaktionsgefäß. Der nicht umgesetzte Überschuß an Alkohol $R_3$—OH wird vom anfallenden Destillat abgetrennt und wiederverwendet. Das Verfahren wird solange fortgesetzt, bis im Destillat kein Alkohol $R_2$—OH mehr anfällt. Das Verfahrensprodukt wird dann aus dem Destillat bzw. dem Rückstand in reiner Form gewonnen.

3. Das Gemisch aus Ausgangsurethan a) und niedrig siedendem Alkohol b) wird in einem Autoklaven auf 120 °C bis 250 °C erhitzt. Nach der Beendigung der Reaktion wird der Inhalt des Autoklaven wie nachstehend beschrieben aufgarbeitet.

4. Eine Lösung des Ausgangsurethans a) im niedrig siedenden Alkohol b) wird kontinuierlich bei 120 bis 250 °C durch einen Reaktor, beispielsweise einen Rohr- oder Schlangenreaktor gepumpt, wobei der Druck im auf die genannte Temperatur erhitzten Reaktor so eingestellt wird, daß ein Verdampfen des Reaktionsgemischs weitgehend unterbleibt. Das den Reaktor verlassende Gemisch wird dann, wie nachstehend beschrieben aufgearbeitet.

Nach der Durchführung des erfindungsgemäßen Verfahrens liegt das Verfahrensprodukt im allgemeinen im Gemisch mit überschüssigem Alkohol $R_3$—OH, abgespaltenem Alkohol $R_2$—OH und gegebenenfalls geringen Mengen an nicht umgesetztem Ausgangsurethan a) vor. Die Isolierung des Verfahrensprodukts aus diesem Gemisch erfolgt im allgemeinen durch Destillation, Extraktion oder Kristallisation. Zwecks einer einwandfreien Aufarbeitbarkeit der beim erfindungsgemäßen Verfahren anfallenden Gemische ist es besonders vorteilhaft und daher bevorzugt, solche Alkohole $R_3$—OH einzusetzen, die zu Verfahrensprodukten führen, deren Siedepunkt mindestens 10 °C, vorzugsweise mindestens 30 °C über oder unter, vorzugsweise über dem Siedepunkt des abgespaltenen Alkohols $R_2$—OH liegen. In einem solchen Falle erfolgt die Aufarbeitung des beim erfindungsgemäßen Verfahren anfallenden Reaktionsgemischs durch eine einfache fraktionierte Destillation, bei welcher der überschüssige Alkohol $R_3$—OH als erste, der abgespaltene Alkohol $R_3$—OH im allgemeinen als zweite oder im Falle von leichter flüchtigem Verfahrensprodukt auch als dritte und das Verfahrensprodukt im allgemeinen als dritte oder auch als zweite Fraktion bzw. als Destillationsrückstand anfallen. Falls auf eine Destillation des Verfahrensprodukts verzichtet wird, oder falls es sich bei dem Verfahrensprodukt um eine nicht destillierbare Substanz handelt, kann seine Reindarstellung gewünschtenfalls durch Extraktion oder Kristallisation entweder mit bzw. in den ohnehin vorliegenden Flüssigkeiten oder unter Verwendung eines Hilfslösungsmittels der nachstehend beispielhaft genannten Art erfolgen. Wegen der im allgemeinen hohen Ausbeuten der erfindungsgemäßen Verfahrens an Verfahrens produkt erübrigt sich oft dessen Abtrennung von gegebenenfalls noch vorliegenden geringen Mengen an Ausgangsurethan a). Dies gilt insbesondere auch für das nach der beispielhaft genannten zweiten Methode des erfindungsgemäßen Verfahrens gegebenenfalls als nicht flüchtiger Rückstand anfallende Verfahrensprodukt.

Im allgemeinen ist die Mitverwendung von Katalysatoren bei der Durchführung des erfindungsgemäßen Verfahrens nicht notwendig. Es hat sich jedoch gezeigt, daß die Mitverwendung von Katalysatoren in einer Menge von 1 ppm bis 10 Gew.-%, vorzugsweise 100 ppm bis 5 Gew.-%, bezogen auf Ausgangsurethan a), oft zu einer Beschleunigung der Umsetzung führt und daher bei einer technischen Durchführung des erfindungsgemäßen Verfharens von Vorteil sein kann.

Geeignete Katalysatoren sind beispielsweise beliebige, als Veresterungskatalysatoren für organische Carbonsäuren bekannte Verbindungen oder auch die bekannten, die Isocyanat-Additionsreaktion beschleunigenden Katalysatoren. Typische Beispiele geeigneter Katalysatoren sind z.B. Lewis-Säuren oder anorganische oder organische Salze von Übergangsmetallen wie z.B. Zinkchlorid, Zinkacetat, Zinkoktoat, Zinnoctoat, Zinn-II-chlorid, Zinn-IV-chlorid, Dimethylzinndilaurat, Kobaltacetat, Kobaltchlorid, Kobaltoktoat, Kupferacetat, Kupferchlorid, Kupfersulfat, Bleiacetat, Bleichlorid, Eisen-IIIchlorid oder Aluminiumchlorid.

Das erfindungsgemäße Verfahren wird im allgemeinen ohne Mitverwendung von Hilfslösungsmitteln durchgeführt. Grundsätzlich ist es jedoch auch möglich, Hilfslösungsmittel wie z.B. Toluol, Xylol, Chlorbenzol, Dichlorbenzol, Sulfolan, ε-Caprolactam, Petrolether, Ethylenglykoldimethylether, Ethylenglykoldiethylether oder Nitrobenzol mitzuverwenden. Derartige Lösungsmittel können auch zur Reindarstellung der Verfahrensprodukte durch Kristallisation bzw. Umkristallisation eingesetzt werden.

Die Verfahrensprodukte entsprechen der Formel

$$R_1\,(NHCOOR_3)_n$$

in welcher

$R_1$, $R_3$ und n die bereits genannte Bedeutung haben.

Die Verfahrensprodukte eignen sich insbesondere zur Herstellung der in ihnen in urethanisierter Form vorliegenden Isocyanate durch eine an sich bekannte thermische Spaltung.

In den nachfolgenden Beispielen beziehen sich alle Angaben in % auf Gewichtsprozente.

Beispiel 1

In einer Kreislaufapparatur können sowohl kontinuierlich als auch diskontinuierlich Urethane höhersiedender Alkohole in Urethane niedrigsiedender Alkohole umgewandet werden.

In einem Reaktor werden 657 g N-Phenyl-O-cyclohexylurethan (3 Mol) vorgelegt, auf ca. 210 °C erwärmt und koninuierlich mit einer Pumpe Methanol aus einem Vorratsgefäß in den Reaktor gepumpt. Überschüssiges Methanol, Cyclohexanol und N-Phenyl-methylurethan destillieren in ein auf 100 °C geheizte Auffanggefäß. Überschüssiges Methanol destilliert in das Vorratsgefäß zurück. Der Sumpf wird destilliert. Aus einem anderen Vorratsgefäß kann das umgesetzte N-Phenyl-O-cyclohexyl-urethan im Reaktor kontinuierlich ersetzt werden.

Nach zwei Stunden ist die Umsetzung beendet. Man erhält nach dem Abdestillieren des Cyclohexanols im Wasserstrahlvakuum 480 g Rückstand der zu 92,3 g % aus N-Phenyl-O-methylurethan besteht (HPLC).

Nach der Destillation erhält man 410 g N-Phenyl-O-methylurethan (= 90 % d. Th.).

Sdp.: 111-113 °C bei 0,26 mbar.

Beispiel 2

1105 g N-Phenyl-O-n-hexylurethan (5 Mol) werden kontinuierlich mit Ethanol in N-Phenylethyl-urethan, wie in Beispiel 1 beschrieben, umgewandelt. Die Temperatur im Auffanggefäß beträgt 120 °C. Nicht umgesetztes Ethanol wird kontinuierlich umgepumpt. Nachdem das Vorratsgefäß und der Reaktor vollständig geleert sind, wird das Reaktionsgemisch fraktioniert destilliert. Ausbeute: 778 g N-Phenyl-O-ethylurethan (= 94 d. Th.).

Sdp.: 152 °C bei 18 mbar

Fp.: 52 °C.

Beispiel 3

1 Mol N-Phenyl-O-cyclohexyl-urethan wurde in 100 ml Methanol warm gelöst und innerhalb von 30 Min. auf ein auf 320 °C geheiztes mit Quarzringen gefülltes 1 m langes Pyrolyserohr von 40 mm lichter Weite aufgepfropft. Die Reaktionsprodukte wurden in 300 ml kaltem Methanol unter Rühren aufgefangen. Nach destillativer Aufarbeitung wurden 147 g O-Methyl-N-phenylurethan in einer Reinheit von 98 % erhalten.

Beispiel 4

748 g 2,4-Bis-(cyclohexoxy-carbonyl-amino)-toluol (2 Mol) werden in 1,5 l Isopropanol gelöst und kontinuierlich in einen auf 220 °C geheizten Schlangenreaktor gepumpt. Der Druck wird auf 10 bar eingestellt und das Reaktionsprodukt kontinuierlich entnommen. Die Verweilzeit kann in weiten Grenzen variiert werden. Aus dem erhaltenen Reaktionsgemisch werden das überschüssige Isopropanol und das Cyclohexanol abdestilliert. Die Ausbeute wird durch Hochdruckflüssigkeitschromatographie (HPLC) bestimmt.

Es werden 602 g Rückstand, der zu 96 % aus 2,4-Bis-(isopropoxy-carbonyl-amino)-urethan besteht, erhalten.

Beispiel 5

368 g 1,6-Bis-(cyclohexoxy-carbonyl-amino)-hexan (1 Mol) werden in 1,5 l Ethanol gelöst und in einem 3 l Autoklaven 2 Studen auf 230 °C erhitzt. Nach dem Erkalten wird das Reaktionsgemisch im Wasserstrahlvakumm von dem überschüssigen Ethanol und entstandenen Cyclohexanol befreit. Das Reaktionsprodukt wird hochdruckflüssigkeitschromatographisch (HPLC) untersucht. Nach Eichung mittel einer authentischen Substanz wird eine Ausbeute von 240 g 1,6-Bis-(ethoxy-carbonyl-amino)-hexan ausgewiesen, entsprechend einer Ausbeute von 92 % d. Th.

Beispiel 6

In einem 1,7 l Autoklaven werden 229 g N-(n-Hexyl)-O-Cyclohexylurethan mit 1 l Methanol zusammengegeben und 1,5 Stunden auf 220 °C erwärmt. Nacht dem Erkalten werden Methanol und Cyclohexanol abgezogen, der Rückstand durch IR, KR und durch HPLC identifiziert und bestimmt.

Ausbeute: 149 g N-(n-Hexyl)-O-methyl-urethan (= 94 % d. Th.).

Beispiel 7

16 Mol O-Cyclohexyl-N-phenylurethan wurden in 16 l Ethanol gelöst und im Autoklaven 1 Stunde auf 230 °C erhitzt. Laut Hochdruckflüssigkeitschromatogramm war das eingesetzte Urethan zu 97 % in O-Ethyl-N-phenylurethan umurethanisiert worden. Der Ansatz wurde destillativ aufgearbeitet. Hierbei fielen 2,5 kg O-Ethyl-N-phenylurethan in 96,5 %iger Reinheit an. in 220 g Rückstand befanden sich 38 % O-Ethyl-N-phenylurethan, 6,2 % N,N'-Diphenylharnstoff und 52,4 % O-Cyclohexyl-N-phenylurethan.

Beispiel 8

486 g (1 Mol) 4,4'-Bis-[(2-Butoxy-)ethoxycarbonylamino] diphenylmethan und 1 Ltr. Ethanol wurde 1 Stunde auf 230 °C unter Druck erhitzt. Nach Abkühlen wurde das Ethanol abdestilliert

und der Rückstand hochdruckflüssigkeitschromatographisch analysiert. Es hatten sich 0,95 Mol 4,4'-Bis-(ethoxycarbonylamino-)diphenylmethan (= 95 % d. Th.) gebildet.

**Ansprüche**

1. Verfahren zur Herstellung von als Ausgangsmaterial zur Herstellung von organischen Isocyanaten durch thermische Spaltung geeigneten Urethanen der Formel

$$R_1(NHCOOR_3)_n$$

in welcher

$R_1$ für einen durch Entfernung der Isocyanatgruppen aus einem n-wertigen organischen Isocyanat mit einem unter Normaldruck oberhalb 100 °C liegenden Siedepunkt erhaltenen organischen, unter den Reaktionsbedingungen inerten Rest,

$R_3$ für einen durch Entfernung der Hydroxylgruppe aus einem unter Normaldruck zwischen 60 und 140 °C siedenden einwertigen Alkohol erhaltenen, unter den Reaktionsbedingungen inerten Rest steht, wobei das dem Rest $R_1$ zugrundeliegende Isocyanat $R_1$ $(NCO)_n$ unter Normaldruck einen mindestens 30 °C höheren Siedepunkt aufweist als der dem Rest $R_3$ entsprechende Alkohol $R_3OH$, und

n für eine ganze Zahl von 1 bis 3 steht,

dadurch gekennzeichnet, daß man Urethane der Formel

$$R_1(NHCOOR_2)_n$$

in welcher

$R_1$ und n die bereits genannte Bedeutung haben und

$R_2$ für einen durch Entfernung der Hydroxylgruppe aus einem einwertigen Alkohol mit einem unter Normaldruck oberhalb 140 °C liegenden Siedepunkt erhaltenen, unter den Reaktionsbedingungen inerten Rest steht,

mit einer solchen Menge eines, unter Normaldruck einen mindestens 10 °C unter dem Siedepunkt des dem Urethan zugrundeliegenden Alkohols $R_2$—OH liegenden Siedepunkt aufweisenden, Alkohols der Formel

$$R_3—OH$$

bei 120 °C bis 400 °C, gegebenenfalls in Gegenwart von Katalysatoren und gegebenenfalls in Gegenwart von Lösungsmitteln zur Reaktion bringt, daß für jedes Mol des Urethans der Formel $R_1$ $(NHCOOR_2)_n$ mindestens n Mole des Alkohols $R_3$—OH zur Verfügung stehen.

2. Verfahren gemäß Anspruch 1, dadurch gekennzeichnet, daß man die Menge des Alkohols $R_3$—OH so bemißt, daß für jedes Mol an Urethan der Formel $R_1$ $(NHCOOR_2)_n$ 5 n bis 20 n Mole Alkohol zur Verfügung stehen.

3. Verfahren gemäß Anspruch 1 und 2, dadurch gekennzeichnet, daß man als Urethane der Formel

$$R_1(NHCOOR_2)_n$$

solche verwendet, für welche

$R_1$ für einen n-wertigen, gegebenenfalls Chlorsubstituierten aliphatischen Kohlenwasserstoffrest mit 6 bis 18 Kohlenstoffatomen, einen gegebenenfalls Methyl- oder Chlorsubstituenten und/oder gegebenenfalls Methylenbrücken aufweisenden aromatischen Kohlenwasserstoffrest mit 6 bis 15 Kohlenstoffatomen, einen cycloaliphatischen Kohlenwasserstoffrest mit 6 bis 15 Kohlenstoffatomen oder einen aliphatischen Kohlenwasserstoffrest mit 7 bis 15 Kohlenstoffatomen steht,

$R_2$ für einen gegebenenfalls $C_1$-$C_4$-Alkoxy- oder $C_1$-$C_4$-Alkoxy-$C_2$-$C_4$-alkoxy-substituierten oder unsubstituierten primären oder sekundären aliphatischen Kohlenwasserstoffrest mit 5 bis 18 Kohlenstoffatomen, einen sukundären cycloaliphatischen Kohlenwasserstoffrest mit 6 bis 15 Kohlenstoffatomen oder einen primären oder sekundären araliphatischen Kohlenwasserstoffrest mit 7 bis 15 Kohlenstoffatomen steht und

n 1 oder 2 bedeutet

und als Alkohole der Formel

$$R_3—OH$$

solche verwendet, für welche

$R_3$ für einen primären oder sekundären aliphatischen Kohlenwasserstoffrest mit 1 bis 4 Kohlenstoffatomen steht.

4. Verwendung der gemäß Anspruch 1-4 erhaltenen Urethane als Ausgangsmaterial zur Herstellung von organischen Isocyanaten.

**Claims**

1. A process for the preparation of urethanes, which are suitable as starting materials for the preparation of organic isocyanates by means of thermal dissociation, of the formula

$$R_1(NHCOOR_3)_n$$

in which

$R_1$ represents an organic radical which is inert under the reaction conditions and is obtained by removing the isocyanate groups from an n-functional organic isocyanate having a boiling point above 100 °C under normal pressure,

$R_3$ represents a radical which is inert under the reaction conditions and is obtained by removing the hydroxyl group from a monohydric alcohol boiling at between 60 and 140 °C under normal pressure, the isocyanate $R_1$ $(NCO)_n$, upon which the radical $R_1$ is based, having a boiling point which is at least 30 °C higher, under normal pressure, than the alcohol $R_3OH$ corresponding to the radical $R_3$, and

n represents an integer from 1 to 3,

characterised in that urethanes of the formula

$$R_1(NHCOOR_2)_n$$

in which

$R_1$ and n have the meaning already given and

$R_2$ represents a radical which is inert under the reaction conditions and is obtained by removing the hydroxyl group from a monohydric alcohol having a boiling point above 140 °C under normal pressure are reacted with such an amount of an alcohol of the formula

$$R_3—OH$$

which has a boiling point under normal pressure at least 10 °C below the boiling point of the alcohol $R_2$—OH upon which the urethane is based, at 120 to 400 °C, optionally in the presence of catalysts and optionally in the presence of solvents, that for each mole of the urethane of the formula $R_1$ $(NHCOOR_2)_n$, at least n mole of the alcohol $R_3$—OH are present.

2. A process according to claim 1, characterised in that the amount of alcohol $R_3$—OH is such that, for every mole of urethane of the formula $R_1$ $(NHCOOR_2)_n$, 5 n to 20 n moles of alcohol are present.

3. A process according to claim 1 and 2, characterised in that the urethanes of the formula.

$$R_1(NHCOOR_2)_n$$

used are those for which

$R_1$ represents an n-functional, optionally chloro-substituted, aliphatic hydrocarbon radical having from 6 to 18 carbon atoms, or an aromatic hydrocarbon radical having from 6 to 15 carbon atoms, which is optionally methyl or chloro-substituted and/or which optionally has methylene bridges, or a cycloaliphatic hydrocarbon radical having from 6 to 15 carbon atoms, or an araliphatic hydrocarbon radical having from 7 to 15 carbon atoms ;

$R_2$ represents an optionally $C_1$-$C_4$ alkoxy or $C_1$-$C_4$ alkoxy $C_2$-$C_4$ alkoxy-substituted or unsubstituted primary or secondary aliphatic hydrocarbon radical having from 5 to 18 carbon atoms, or a secondary cycloaliphatic hydrocarbon radical having from 6 to 15 carbon atoms or a primary or secondary araliphatic hydrocarbon radical having from 7 to 15 carbon atoms and

n represents 1 or 2

and the alcohols of the formula

$$R_3—OH$$

used are those for which

$R_3$ represents a primary or secondary aliphatic hydrocarbon radical having from 1 to 4 carbon atoms.

4. The use of the urethanes obtained according to claims 1 to 4, as starting materials for the preparation of organic isocyanates.

**Revendications**

1. Procédé de production d'uréthannes pouvant être utilisés comme matière de départ pour la production d'isocyanates organiques par décomposition thermique, de formule

$$R_1(NHCOOR_3)_n$$

dans laquelle

$R_1$ désigne un reste organique inerte dans les conditions réactionnelles, obtenu par élimination des groupes isocyanate d'un isocyanate organique de valence n de point d'ébullition supérieur à 100 °C à la pression normale,

$R_3$ est un reste inerte dans les conditions réactionnelles, obtenu par élimination du groupe hydroxyle d'un alcool monovalent bouillant entre 60 et 140 °C à la pression normale, l'isocyanate $R_1$ $(NCO)_n$ à la base du reste $R_1$ présentant à la pression normale un point d'ébullition supérieur d'au moins 30 °C à celui de l'alcool $R_3OH$ correspondant au reste $R_3$, et

n est un nombre entier de 1 à 3,

caractérisé en ce qu'on fait réagir des uréthannes de formule

$$R_1(NHCOOR_2)_n$$

dans laquelle

$R_1$ et n ont la définition déjà mentionnée et

$R_2$ désigne un reste inerte dans les conditions réactionnelles, obtenu par élimination du groupe hydroxyle d'un alcool monovalent de point d'ébullition supérieur à 140 °C à la pression normale,

avec une quantité d'un alcool présentant à la pression normale un point d'ébullition inférieur d'au moins 10 °C au point d'ébullition de l'alcool $R_2$—OH à la base de l'uréthanne, de formule

$$R_3—OH$$

à 120-400 °C, éventuellement en présence de catalyseurs et, le cas échéant, en présence de solvants, chosie de manière qu'au moins n moles de l'alcool $R_3$—OH soient disponibles pour chaque mole de l'uréthanne de formule $R_1(NHCOOR_2)_n$.

2. Procédé suivant la revendication 1, caractérisé en ce qu'on mesure la quantité d'alcool $R_3$-OH de manière que 5 n à 20 n moles d'alcool soient disponibles pour chaque mole d'uréthanne de formule $R_1(NHCOOR_2)_n$.

3. Procédé suivant les revendications 1 et 2, caractérisé en ce qu'on utilise comme uréthannes de formule

$$R_1(NHCOOR_2)_n$$

des uréthannes pour lesquels

$R_1$ est un reste d'hydrocarbure aliphatique de valence n, éventuellement substitué par du chlore, ayant 6 à 18 atomes de carbone, un reste

d'hydrocarbure aromatique ayant 6 à 15 atomes de carbone, portant éventuellement des substituants méthyle ou chloro et/ou, le cas échéant, des ponts méthyléniques, un reste d'hydrocarbure cycloaliphatique ayant 6 à 15 atomes de carbone ou un reste d'hydrocarbure araliphatique ayant 7 à 15 atomes de carbone,

$R_2$ désigne un reste d'hydrocarbure aliphatique primaire ou secondaire ayant 5 à 18 atomes de carbone, non substitué ou éventuellement substitué avec des groupes alkoxy en $C_1$ à $C_4$ ou (alkoxy en $C_1$ à $C_4$)-alkoxy en $C_2$ à $C_4$, un reste secondaire d'hydrocarbure cycloaliphatique ayant 6 à 15 atomes de carbone ou un reste primaire ou secondaire d'hydrocarbure aliphatique ayant 7 à 15 atomes de carbone et

n est égal à 1 ou 2

et on utilise comme alcools de formule

$$R_3—OH$$

des alcools pour lesquels

$R_3$ est un reste primaire ou secondaire d'hydrocarbure aliphatique ayant 1 à 4 atomes de carbone.

4. Utilisation des uréthannes obtenus suivant les revendications 1 à 4 comme matière de départ pour la production d'isocyanates organiques.